# EUROPEAN PATENT SPECIFICATION

(11) **EP 3 166 632 B1**
(45) Date of publication and mention of the grant of the patent: **11.12.2019**
(21) Application number: 15735934.0
(22) Date of filing: 07.07.2015
(51) Int. Cl.: A61K 39/02, C12N 9/10, C12N 9/12

(54) **MODIFIED BACTERIA FOR IMPROVED VACCINES AGAINST BRUCELLOSIS**
MODIFIZIERTE BAKTERIEN FÜR VERBESSERTE IMPFSTOFFE GEGEN BRUCELLOSE
BACTÉRIES MODIFIÉES POUR VACCINS AMÉLIORÉS CONTRE LA BRUCELLOSE

(30) Priority: 10.07.2014 EP 14306125; 17.04.2015 EP 15305578
(43) Date of publication of application: 17.05.2017
(73) Proprietor: INSERM (Institut National de la Santé et de la Recherche Médicale), 75013 Paris (FR); Centre National de la Recherche Scientifique (CNRS), 75016 Paris (FR); Université d'Aix-Marseille, 13007 Marseille (FR); Universidad de Navarra, 31009 Pamplona (Navarra) (ES)
(72) Inventor: GORVEL, Jean-Pierre, F-13288 Marseille Cedex 09 (FR); ARCE-GORVEL, Vilma, F-13288 Marseille Cedex 09 (FR); HANNIFFY, Sean, F-13288 Marseille (FR); CONDE-ALVAREZ, Raquel, E-31009 Pamplona (ES); MORIYON, Ignacio, E-31009 Pamplona (ES); IRIARTE, Maite, E-31009 Pamplona (ES); ZUNIGA-RIPA, Amaia, E-31009 Pamplona (ES)
(74) Representative: Plasseraud IP
(86) International application number: PCT/EP2015/065478
(87) International publication number: WO 2016/005390

(56) References cited:
- WO-A1-2011/033129
- WO-A1-2012/131128
- RAQUEL CONDE-ÁLVAREZ ET AL: "The Lipopolysaccharide Core of Brucella abortus Acts as a Shield Against Innate Immunity Recognition", PLOS PATHOGENS, vol. 8, no. 5, 10 May 2012 (2012-05-10), page e1002675, XP055159517, DOI: 10.1371/journal.ppat.1002675 cited in the application
- A. ZUNIGA-RIPA ET AL: "Brucella abortus Depends on Pyruvate Phosphate Dikinase and Malic Enzyme but Not on Fbp and GlpX Fructose-1,6-Bisphosphatases for Full Virulence in Laboratory Models", JOURNAL OF BACTERIOLOGY, vol. 196, no. 16, 16 June 2014 (2014-06-16), pages 3045-3057, XP055160306, US ISSN: 0021-9193, DOI: 10.1128/JB.01663-14
- MARÍA-JESÚS GRILLÓ ET AL: "What have we learned from brucellosis in the mouse model?", VETERINARY RESEARCH, BIOMED CENTRAL LTD, LONDON, UK, vol. 43, no. 1, 13 April 2012 (2012-04-13) , page 29, XP021132282, ISSN: 1297-9716, DOI: 10.1186/1297-9716-43-29
- BLASCO JOSÉ M ET AL: "Control and eradication of Brucella melitensis infection in sheep and goats", THE VETERINARY CLINICS OF NORTH AMERICA, FOOD ANIMAL PRACTICE, USA, vol. 27, no. 1, 1 March 2011 (2011-03-01), pages 95-104, XP009181801, ISSN: 1558-4240, DOI: 10.1016/J.CVFA.2010.10.003 cited in the application

## Description

### Field of the invention

The present invention relates to modified bacteria of the genus *Brucella* for use as vaccines against brucellosis.

### Background of the invention

Brucellosis is a bacterial zoonosis caused by *Brucella,* a genus of gram-negative bacteria that behave as facultative intracellular pathogens of ruminants, suids, canids, camelids and several forms of wildlife. These bacteria are extremely infectious, and humans acquire brucellosis readily from animals and their products. There are several highly homologous *Brucella* species (Moreno et al., Vet.Microbiol.90,209-227,2002; Chain et al., Infect.Immun. 73,8353-8361,2005) among which *B. abortus* preferentially infects cattle, *B. melitensis* sheep and goats, and *B. suis* swine and wildlife. These three species are the main cause of human brucellosis. Sheep may also be infected by *B. ovis.*

Brucellosis is a main cause of abortions and infertility in animals and, therefore, of heavy economic losses. In cattle, a 50% decrease in production (meat, milk, costs of culling) is often given as an estimate. The number of domestic animals susceptible to brucellosis in the world is estimated at around 4.000.000.000 and, of this number, less than 20% are in countries free of the disease (Anonymous source, 2006). The incidence of brucellosis is very high in countries at the South and East European Union borders (Seimenis et al., Ann.Ist.Super.Sanita. 42, 437-445,2006) and in low-income countries throughout the world. In the USA, the Northern European Union area and France, success has been achieved in eradicating the bovine disease after heavy investments and many years of vaccination and culling. *B. melitensis* infection has proved more intractable, largely because of the difficulties of animal management under the extensive breeding conditions of semiarid areas.

Human brucellosis is always a consequence of animal brucellosis and is most often caused by *B. melitensis.* If untreated, human brucellosis leads to severe invalidating consequences and is deadly in about 3% of cases. Because of the deficiencies in Health Services of most countries where the disease is endemic, there are no reliable data on the global incidence of the human form of the disease. Incidence figures vary widely (from <0.01 to >200 per 100,000), reflecting the difficulties in recognizing a disease that, although grave, lacks pathognomonic symptoms and is thus underreported (Corbel, Emerg.Infect.Dis. 3, 213-221,1997). A figure of 500.000 new cases/year is usually given as an estimate (Pappas et al., Lancet Infect.Dis. 6, 91-99,2006), and recent data from the emerging economies in the Near East and Asia confirm a very high incidence of the disease in humans wherever diagnosis is implemented (for example 15.000 new cases/year in Iran, or an incidence of almost 1400 cases per 100 000 inhabitants in Northern China) (Anonymous, Iranian Journal of Microbiology, 5:183.1,2013). Because of all these circumstances, the World Health Organisation has classified brucellosis among the top seven "neglected zoonoses", a group of diseases that are simultaneously a threat to human health and a cause of the perpetuation of poverty (Maudlin and Weber, Report of a meeting in Geneva, 2006).

Vaccination is critical to control and eradicate ruminant brucellosis (Nicoletti, CRC Press, Boca Raton., 283-299,1990; Garin-Bastuji et al., Vet.Res. 29, 255-274, 1998). Since there is no human vaccine, this is also the best way to lessen human infections. Vaccines *B. abortus* S19 and *B. melitensis* Rev 1 are effective against *B. abortus* in cattle and *B. melitensis* in goats and sheep. However, these classical vaccines do not provide 100% protection, are virulent for humans and, in the case of Rev 1, streptomycin-resistant (one of the antibiotics of choice to treat brucellosis) (Garin-Bastuji et al., Vet.Res. 29, 255-274, 1998 ; Ariza et al., PLoS.Med. 4, e317,2007). Moreover, they are abortifacient in pregnant animals. This last disadvantageous trait is very conspicuous with the vaccine Rev 1, and seriously hampers the application of mass-vaccination strategies that are essential for brucellosis control in small ruminants in most areas of the world (Blasco and Molina-Flores, Vet.Clin.North Am.Food Anim Pract. 27:95-104,2011). Therefore, there is a need to develop safer and more efficient vaccines against brucellosis. *Brucella* has the ability to avoid detection by innate immunity at the onset of infection, thus retarding an adaptive cellular response and making possible for the pathogen to reach sheltered intracellular niches. This ability is related to the failure of innate immunity pathogen-recognition receptors (PRRs) to identify the *Brucella* surface molecules that normally bear the cognate pathogen-associated molecular patterns (PAMP) (Barquero-Calvo E et al., PLoS ONE 2: e631, 2007), and in particular the lipopolysaccharide (LPS) of these strains. These *Brucella* molecules are, therefore, targets for developing more effective vaccines. LPS, one of the most conspicuous PAMP-bearing molecules of the gram-negative surface, is a glycolipid made of three consecutive sections: lipid A, core oligosaccharide and the polysaccharide O side chains (O-PS). Comparative studies have established a clear role for *Brucella* lipid A in virulence (Lapaque et al., 2005). Similarly, the *Brucella* LPS O-PS is critical in virulence but its removal generates over-attenuation and poor vaccines (R form of *Brucella*) (González et al., 2008; Barrio et al., 2009). WO2011/033129 discloses a new vaccine against brucellosis based on a modified *Brucella* carrying an inactivated gene (*wadC*) coding for a glycosyltransferase involved in the synthesis of the LPS core. The disruption of *wadC* removes part of the core without affecting the O-PS. The analysis of the biological properties of a *wadC* mutant of *B. abortus* (BABΔ*wadC*) has been published (this strain was previously described as BaΔwadC by Conde-Alvarez et al., PLoS Pathog., 8(5),e1002675,2012). BABΔ*wadC* is unable to multiply in bone marrow derived macrophages (BMDM) and triggers a strong IL-12 response in these cells, which results in a protective Th1 response. BABΔ*wadC* provides a protection against *B. abortus* that is similar to that obtained with the S19 vaccine (Conde-Álvarez et al., PLoS Pathog., 8(5),e1002675,2012). Thus, the BABΔ*wadC* vaccine against *Brucella* is as efficient as S19 whilst carrying fewer mutations than that described for S19.

Thus, the BABΔ*wadC* mutant represents a great improvement in the field of vaccines against brucellosis, but there is still a need to develop new and safer vaccines with higher efficacy against this disease.

### Summary of the invention

The inventors have elaborated a set of metabolic mutants of *Brucella* contained within the glycolytic/gluconeogenic and Krebs cycle pathways, and used one of these mutants as a vaccine against brucellosis. The mutated bacterium of the genus *Brucella* of the invention carries an inactivated gene coding for a glycosyltransferase involved in the synthesis of the core of the LPS (BABΔ*wadC*) and an inactivated gene coding for an enzyme involved in several metabolic pathways such as Krebs cycle and gluconeogenesis: the pyruvatephosphate dikinase (ppdK: ORF BAB1_0525; or homologues). Accordingly, the present invention relates to the mutant BABΔwadCΔppdK.

The inventors found that such a BABΔwadCΔppdK mutant shows a normal intracellular trafficking but is defective in intracellular multiplication once it has reached the replication niche. The combination of mutations in genes ppdK and wadC generates highly effective vaccines against brucellosis in the brucellosis mouse model.

Therefore, the present invention relates to a modified bacterium belonging to the *Brucella* genus that carries a double mutation comprising:
- an inactivated *wadC* gene, and
- an inactivated ppdK gene.

The invention also relates to a vaccine against brucellosis in human and/or animals comprising said modified bacterium belonging to the *Brucella* genus that carries an inactivated *wadC* gene and an inactivated *ppdK* gene.

### Detailed description of the invention

An aspect of the invention concerns a bacterium of the genus *Brucella* carrying an inactivated *wadC* gene and an inactivated *ppdK* gene.

According to the invention, by "inactivated gene" it is meant a gene that encodes either a non-functional protein or no protein at all. According to the invention the inactivation of a gene can be carried out by the various methods known by the skilled person. Examples of methods for inactivating a gene are the knock out, particularly by directed mutagenesis or homologous recombination, as described in Conde-Álvarez R. et al., Cell Microbiol 2006 Aug;8(8):1322-35. A particular method for inactivating a gene according to the invention is described in the experimental section.

By "wadC" it is meant the *wadC* gene of a bacterium of the *Brucella* genus, including *B. abortus.* The *wadC* gene of *B. abortus* has been described in WO2011/033129 as "BABLpcC" and has the sequence corresponding to "SEQ ID NO:1" in this PCT application.

*wadC* is a gene coding for a glycosyltransferase involved in the synthesis of the core of the LPS in *Brucella.*

The bacterium of the invention carries an inactivated *wadC* gene. Therefore, and similarly to the mutant BABΔwadC presented in WO2011/033129, the bacterium of the invention presents a LPS which lacks part of its core but keeps an intact O-chain. This property allows the mutant bacterium of the invention to trigger a more intense and protective immune response than the commonly available/utilised vaccines against *Brucella* (S19, rev1). The inactivation of *wadC* has been described in WO2011/033129 and is similarly performed in the present application. Examples of *wadC* genes present among *Brucella* species include those that are presented hereinafter.

| ***Brucella* species** | **wadC gene ID** |
|---|---|
| *Brucella abortus* | Gene ID: 3788779 |
| *Brucella melitensis* | Gene ID: 1196220 |
| *Brucella suis* | Gene ID: 1167186 |
| *Brucella ovis* | Gene ID: 5203447 |
| *Brucella pinnipedialis* | Gene ID: 10998034 |
| *Brucella ceti* | Gene ID: 17713240 |
| *Brucella microti* | Gene ID: 8321077 |
| *Brucella canis* | Gene ID: 5785277 |

By "*ppdK*" it is meant the *ppdK* gene of a bacterium of the *Brucella genus*, including *B. abortus. ppdK* is a gene coding for an enzyme involved in metabolic pathways in *Brucella* (such as the Krebs cycle and gluconeogenesis): the phosphoenolpyruvate dikinase or pyruvate phosphate dikinase (*ppdK* of *B. abortus*: ORF BAB1_0525). *ppdK* is necessary for the efficient intracellular multiplication of *B. abortus* in cultured macrophages (Zúñiga-Ripa et al., J Bact, doi:10.1128/JB.01663-14).

Examples *ppdK* genes present among *Brucella* species include those that are presented hereinafter:

| ***Brucella* species** | ***ppdK* gene (or homologous name) ID** |
|---|---|
| *Brucella abortus* | Gene ID: 3788861 |
| *Brucella melitensis* | Gene ID: 1197147 |
| *Brucella suis* | Gene ID: 12136820 |
| *Brucella ovis* | Gene ID: 5201697 |
| *Brucella pinnipedialis* | Gene ID: 10997008 |
| *Brucella ceti* | Gene ID: 17714040 |
| *Brucella microti* | Gene ID: 8320067 |
| *Brucella canis* | Gene ID: 5784717 |

The bacterium according to the invention is inactivated for *wadC* and *ppdK.*

The BABΔwadCΔppdK mutant shows normal intracellular trafficking and is only defective in intracellular multiplication once it reaches the replicating niche. The inventors have discovered that a combination of mutations in genes ppdK and wadC generates highly effective vaccines in BALB/c mice. Mice vaccinated with the double BABΔwadCΔppdK mutant were as protected against the virulent 2308 challenge as those vaccinated with S19 or BABΔwadC but, in contrast, contained almost no residual vaccine presence in their spleens at two weeks post- challenge (see following table). Current vaccines cannot be safely applied in adult animals because their extended period of permanence in the host implies a high risk of vaccine-induced abortion and milk excretion. Therefore the short period of permanence exhibited by the BABΔwadCΔppdK mutant increases the safety and animal age-range for use as brucellosis vaccines. Furthermore, it is expected that a short permanence reduces the interference of the new vaccine in serological diagnostic tests, a drawback of classical vaccines (i.e. S19 and Rev 1), and this will thus contribute to solving the DIVA problem (see hereinafter).

The following table represents protection against *B. abortus* infection in BALB/c mice obtained by vaccination with BABΔwadC, BABΔwadCΔppdK or *B. abortus* S19.

| | X log₁₀ CFU in spleen ± SD at post-challenge week | | | |
|---|---|---|---|---|
| | 2 | | 6 | |
| Vaccine | *B. abortus* 2308 | Vaccine | *B. abortus* 2308 | Vaccine |
| S19 | 1.30 ± 0.35 | 4.06 ± 1.19 | 3.59 ± 1.40 | 2.99 ± 0.15 |
| *BABΔwadC* | 1.58 ± 0.04 | 3.90 ± 0.07 | 4.44 ± 0.18 | 2.88 ± 0.13 |
| *BABΔwadCΔppdK* | 2.07 ± 1.17 | 0.49 ± 0.04 | 3.89 ± 1.94 | 0.77 ± 0.22 |
| Saline | 6.14 ± 0.25 | | 6.07 ± 0.22 | |

Mice vaccinated with the modified bacterium according to the invention show a higher immune response than the one obtained with mice vaccinated with the single mutants BABΔwadC or BABΔppdk.

When administered to a human or an animal, the mutant according to the invention triggers an intense release of several inflammatory interleukins, particularly of IL6, IL12 and TNFα The specific combination of mutations in the ppdK and wadC genes in a bacterium according to the invention generates a strong immune response and thereby enhances the efficiency of a vaccine containing such mutant.

Thus, the inventors demonstrated that surprisingly, there is a synergic effect between the mutations of ppdK and wadC.

In a particular embodiment, the invention also concerns a bacterium according to the invention further comprising a mutation that enables to readily differentiate the mutant of the invention from the wild type bacterium.

One of the main drawbacks of current *Brucella* vaccines is the commonly termed "DIVA" problem (differentiation of infected and vaccinated animals). The most effective brucellosis diagnostic tests are those that detect antibodies to the O-PS section of the outer membrane LPS. However, like infection by wild type strains, vaccination with Rev 1 or S19 triggers antibodies to the O-PS which thus hinders the differentiation of infected and vaccinated animals. Conjunctival vaccination of ruminants during the first months of life partially solves this problem. However, this is not compatible with mass vaccination programs. While the use of mutants devoid of the O-PS (the R mutants) has been proposed as a solution, it has been proved that vaccines deleted in the O-PS are considerably less efficacious than the classical vaccines (González et al., PLoS ONE 3, e2760,2008; Barrio et al., Vaccine, 27(11):1741-9,2009).

The *Brucella* S-LPS O-polysaccharide is a homopolymer of N-formyl-perosamine that occurs either exclusively as α (1-2) linkages or as a combination of α (1-2) plus α (1-3) linkages in a > 4:1 proportion (Perry and Bundle, Adams LG, ed. Advances in brucellosis research. College Station: Texas A&M University Press, 76-88, 1990). This O-polysaccharide carries three basic types of overlapping epitopes: C (common to all chemical types of *Brucella* O-polysaccharide); M (present in those O-polysaccharides with α (1-3) linkages) and A (present in those O-polysaccharides with no α (1-3) linkages or with a proportion of α (1-2) to α (1-3) linkages higher than 4:1). In all these epitopes, the formyl group plays an essential role (Bundle et al, Infect Immun., 57(9):2829-36 1989).

Therefore, a further aspect of the invention is to provide a *Brucella* mutant according to the invention which triggers an immune response wherein the antibodies triggered against the O-PS of the LPS of the modified bacterium are different from those triggered against the O-PS of the LPS of the wild type bacterium. Hence, the antibodies triggered by the vaccine according to the invention are easily detected and differentiated from the antibodies triggered by the wild type bacterium, thereby allowing the differentiation of vaccinated and infected animals. For this purpose, the inventors have added an antigen tag on to the O-PS of the LPS of the modified bacterium according to the invention. This addition is achieved by the expression of an heterologous enzyme targeting the O-PS in the modified bacteria according to the invention.

Thus, in a particular embodiment, the bacterium according to the invention further expresses a heterologous enzyme that acylates, typically this heterologous enzyme acetylates the O-PS of the LPS.

More particularly, the said heterologous enzyme acylates the perosamine of the O-PS.

The said heterologous enzyme is capable of transferring on to the O-PS an acyl group which is not formyl, typically an acetyl group; such a group can include 3-deoxi-L-glycerotetronyl, 33-hydroxypropionyl, S(+)2-hydroxypropionyl and R(-)2-hydroxypropionyl.

Thus, in a particular embodiment, the bacterium according to the invention can express a heterologous enzyme that is a N-acyltransferase, and typically, the said heterologous enzyme is a N-acetyltansferase (Enzyme Commission number 2.3).

Preferably, the said N-acetyltransferase is expressed by the wbdR gene of *E.coli* O:157:H7 (Gene ID: 962088).

Hence, in a further embodiment the modified bacterium of the invention is a BABΔwadCΔppdK::wbdR mutant.

Methods for introducing heterologous genes in a bacterium are well known by the one skilled in the art.

*E. coli* O:157:H7 O-PS contains only one N- acetyl-perosamine unit in a repeating tetrasaccharide and cross-reacts only weakly with *Brucella.*

In the natural hosts, the tagging with the use of the *wbdR* gene of *E. coli* O:157:H7 is only observed clearly in animals immunized with killed bacteria in Freund's adjuvant, and infection triggers only transient IgM antibodies. The modified *Brucella-wbdR* construct according to the invention shows a new epitope not existing in the original strain, and antibodies to this new epitope are generated upon infection in laboratory animals. These *Brucella-wbdR-specific* antibodies according to the invention can then be detected for instance in an ELISA test, which would be one of the possible formats of a test to differentiate infected from vaccinated animals.

The invention also concerns the therapeutic applications of the bacterium mutant according to the invention. In particular, the invention relates to the modified *Brucella* according to the invention for use as a vaccine of the human or animal body.

Indeed, the inventors have shown that the modified bacteria according to the invention can be used as live vaccines.

The invention thus relates to the modified bacterium of the *Brucella* genus according to the invention, for use in a method for vaccinating the human or animal body against a disease caused by the wild type of said modified *Brucella.*

In a further aspect of the invention, this disease is brucellosis.

The invention also relates to a method for vaccinating a subject against a disease caused by a bacterium of the genus *Brucella,* said method comprising the step of administering to a subject in need thereof a therapeutically effective amount of said modified *Brucella* bacterium according to the invention.

In a further aspect of the invention, the bacterium of the *Brucella* genus is selected from *Brucella abortus, Brucella melitensis, Brucella suis, Brucella ovis, Brucella pinnipedialis*, *Brucella ceti*, *Brucella microti*, and *Brucella canis.*

Preferably, the bacterium is selected from *Brucella abortus*, *Brucella melitensis*, more preferably the bacterium is of the *Brucella abortus* species.

When the bacterium according to the invention is of the *Brucella* abortus species, then it can be used as a universal vaccine against brucellosis.

By "universal vaccine against brucellosis", it is meant a vaccine that is efficient against all species of bacteria of the genus *Brucella.*

By "efficient" it is meant that can significantly reduce a subsequent infection caused by a bacterium of the genus *Brucella.*

The inventors found that, surprisingly, a vaccine comprising a modified bacterium according to the invention, wherein said modified bacterium is of the *Brucella abortus* species, can be used to vaccine a human or animal against all species of bacteria belonging to the genus *Brucella.*

As used herein, "subject" refers to a human or animal that may benefit from the administration of a modified bacterium as recited herein.

In a preferred embodiment the subject is an animal selected from the group of cattle, sheep, goats and swine.

The following table represents the association of the different species of *Brucella* with their preferred host.

| ***Brucella* species** | **Preferred host** |
|---|---|
| *Brucella abortus* | Cattle |
| *Brucella melitensis* | Sheep, Goats |
| *Brucella suis* | Swine, hare and wild rodents |
| *Brucella ovis* | Sheep |
| *Brucella pinnipedialis* | Cetaceans, Seals |
| *Brucella ceti* | Cetaceans, Seals |
| *Brucella microti* | Common vole (*Microtus arvalis*) |
| *Brucella canis* | Canids |

By a "therapeutically effective amount" of a modified *Brucella* bacterium as described previously, is meant a sufficient amount to treat the disease, at a reasonable benefit/risk ratio applicable to any medical treatment.

A vaccine is defined herein as a biological agent which is capable of providing a protective response in an animal or a human to which the vaccine has been delivered and is incapable of causing severe disease. The vaccine stimulates antibody production or cellular immunity against the pathogen causing the disease; administration of the vaccine thus results in immunity from the disease.

If desired, the live vaccines according to the invention may contain an adjuvant. Examples of suitable compounds and compositions with adjuvant activity are well known in the art. Furthermore, nucleic acid sequences encoding polypeptides for pharmaceutical or diagnostic applications, in particular immunomodulators such as lymphokines, interferons or cytokines, may be incorporated into the vaccine.

A vaccine according to the invention can be prepared by conventional methods such as those commonly used for the commercially available live attenuated vaccines.

The vaccine may be administered by intramuscular, intradermal, subcutaneous or intranasal inoculation or by injection in an amount which is effective to protect the subject against challenge by a virulent strain of *Brucella.* This amount may vary according to the subject being inoculated, taking into consideration the size and weight of the subject. The vaccine according to the invention comprises an effective dosage of the *Brucella* mutant as the active component, i.e. a sufficient amount of *Brucella* mutant that will induce immunity in the vaccinated animals, against challenge by the virulent *Brucella.*

Immunity is defined herein as the induction of a significant higher level of protection in a population of animals or humans against mortality and/or clinical symptoms after vaccination compared to an unvaccinated group. In particular, the vaccine according to the invention prevents a large proportion of vaccinated subjects against the occurrence of clinical symptoms of the disease and/or mortality. When providing a subject with live bacteria vaccines, the dosage of administered bacteria will vary depending upon such factors as the route of administration, subject's species, age, weight, height, sex, general medical condition, previous medical history, etc. In general, it is desirable to provide the recipient with a dosage of the above bacteria ranging from about 10⁵ cfu/kg to 10⁸ cfu/kg (body weight of subject), although a lower or higher dosage may be administered.

In addition to the *Brucella* mutant (or described variants thereof), the invention can also include combination vaccines comprising a vaccine strain capable of inducing protection against another pathogen.

Further aspects and advantages of this invention will be disclosed in the following figures and examples, which should be regarded as illustrative and not limiting the scope of this application.

### Brief description of the figures

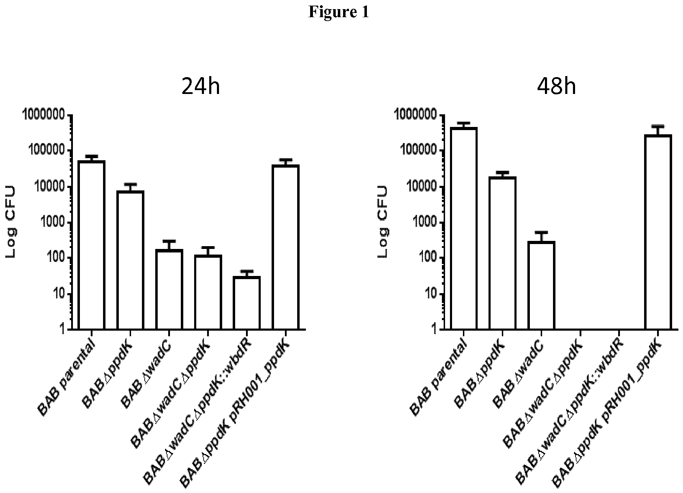

**Figure 1****:** Intracellular replication of the Ba-parental strain and the construct strains BABΔ*wadC*, BABΔ*ppdK, BABΔppdK pRH001_ppdK,* BABΔ*wadC*Δ*ppdK* and *BABΔwadCΔppdK*::*wbdR* in BMDC at 24h and 48h post-infection. The results are representative of the mean ± standard error of three independent experiments, and each experiment was performed in triplicate.

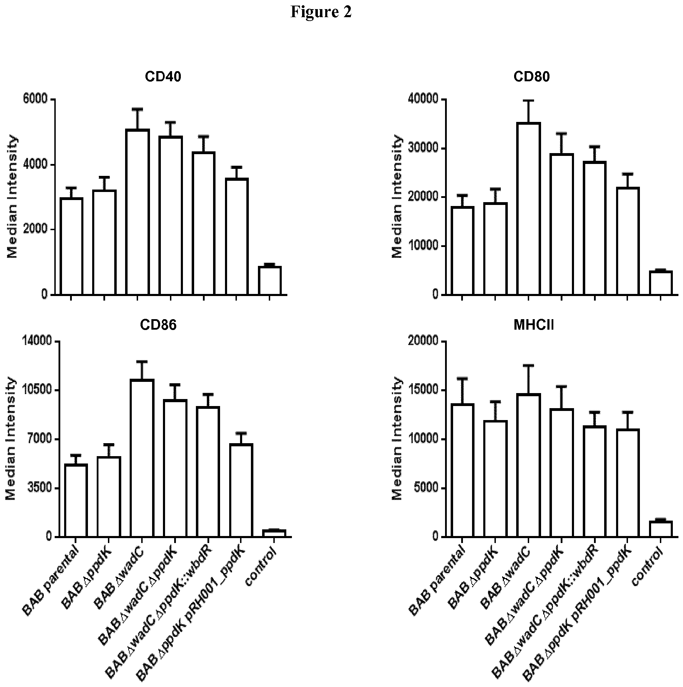

**Figure 2****:** Surface expression of activation molecules CD40, CD80, CD86 and MHCII in BMDC at 24h post-infection with the Ba-parental, BABΔ*wadC*, BABΔ*ppdK, BABΔppdK pRH001_ppdK,* BABΔ*wadC*Δ*ppdK* and *BABΔwadCΔppdK*::*wbdR* strains as measured by flow cytometry. The results are representative of the mean median intensity ± standard error and are representative of four independent experiments.

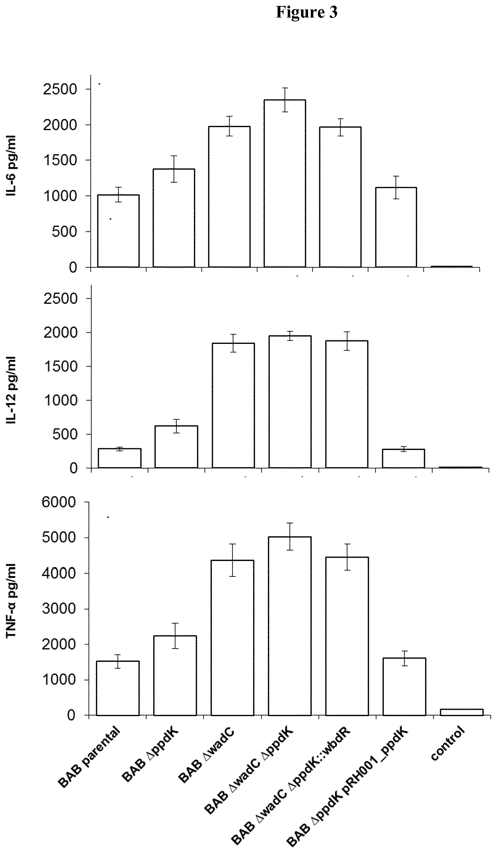

**Figure 3****:** IL-6, IL-12 p70 and TNF-α released by BMDC at 24 h after infection with the Ba-parental, BABΔ*wadC*, BABΔ*ppdK, BABΔppdK pRH001_ppdK,* BABΔ*wadC*Δ*ppdK* and BABΔ*wadCΔppdK*::*wbdR* strains as measured by ELISA. Results are representative of four independent experiments.

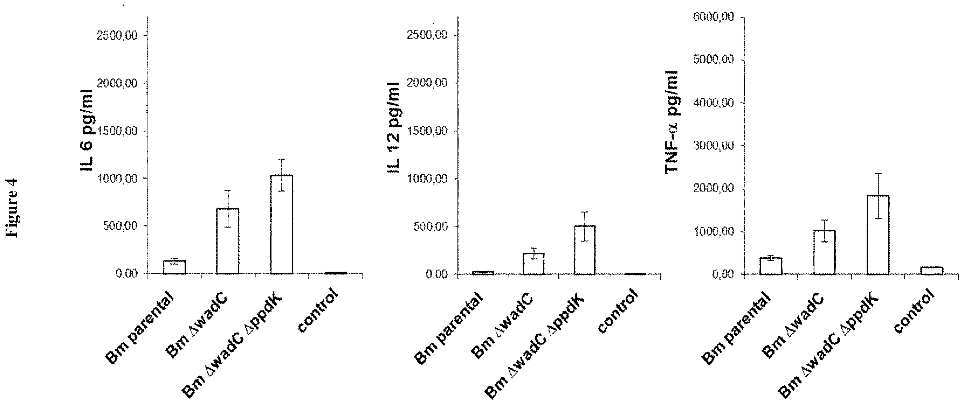

**Figure 4****:** IL-6, IL-12 and TNF- α released by BMDC at 24 h after infection with the Bm-parental, BmΔ*wadC*, BmΔ*wadCΔppdk* strains as measured by ELISA.

### EXAMPLES

### 1) Construction of wadC mutant (BABΔwadC)

BABΔwadC has already been described in WO2011/033129 under the name of mutant BABLpcC with an inactivated *lpcC* gene and is similarly performed in the present invention.

### 2) Construction of mutants in gene ppdK encoding the pyruvate phosphate dikinase (BABΔppdK)

Mutation of gene *ppdK* (ORF BAB1_0525; or homologues) was constructed by PCR overlap on *B. abortus* 2308 as follows: Primers *ppdK-*F1(5'-CTCCCGATTCATTTTTCACG-3'SEQ ID No:1) and *ppdK-*R2 (5'-TGCTCATTTCAGCCAGGTT-3' SEQ ID No:2) were used to amplify a 288-bp fragment including the first 103 bp of the *ppdK* ORF, as well as 185 bp upstream of the *ppdK* start codon and primers *ppdK*-F3 (5'-AACCTGGCTGAAATGAGCACGGGTCTCGACTATGTGTCC-3' SEQ ID No:3) and *ppdK-*R4 (5'-TCAACGCATCAAAGCAGAAG-3' SEQ ID No:4) to amplify a 220 bp including the last 86 bp of the *ppdK* ORF and 134 bp downstream of the *ppdK* stop codon.

Both fragments were ligated by overlapping PCR using primers *ppdK*-F1 and *ppdK-*R4 and the fragment obtained, containing the deletion allele, was cloned into pCR2.1 to generate pMZI-1, was sequenced to confirm that the reading frame had been maintained, and was subcloned into pJQKm to produce the mutator plasmid pMZI-2. This plasmid was introduced into *B. abortus* 2308 and both the deletion mutant (BABΔppdK) and the sibling revertant strain generated by allelic exchange were selected by Nal and sucrose resistance and Km sensitivity, and by PCR using *ppdK-*F1 and *ppdK-*R4 which amplified a fragment of 508 bp in BABΔppdK and a fragment of 2983 bp in BABppdK-sibling revertant strain. The generated mutation resulted in a 93% loss of the *ppdK* orf, and complementation with a plasmid carrying the wild type gene (strain BABΔppdK pRH001_ppdK) confirmed that the phenotype of the mutant was caused by the deletion in *ppdK.*

### 3) Construction of a double mutant according to the invention: BABΔwadCΔppdK

To construct the BABΔwadCΔppdK double mutant, the mutator plasmid pMZI-2 was introduced in to strain BABΔwadC carrying the *wadC* mutation. After allelic exchange, the double mutant was selected as described above using primers *ppdK-*F1 (SEQ ID No:1) and *ppdK*-R4 (SEQ ID No:4).

The BmΔwadCΔppdK double mutant has been contructed similarly but by using a *Brucella* melitensis strain instead

### 4) Genetic tagging of Brucella vaccine candidates.

Using a non-integrative plasmid, the *wbdR* (ORF z3192) gene encoding the transferase that acetylates perosamine in the heteropolymeric LPS O-polysaccharide of *E. coli* O:157:H7 was cloned and the plasmid carrying this introduced into *B. abortus* by conjugation. 1H-NMR analysis demonstrated that this construct expresses an O-polysaccharide containing 60% N-acetylperosamine and 40% formylperosamine. Monoclonal antibody analysis showed that the construct is devoid of the A epitope while keeping the C epitope. In addition, the N-acetylperosamine-N-formylperosamine polysaccharide carried a new epitope(s) not present in wild type *B. abortus*, as demonstrated by immunization of laboratory animals.

To overcome any possible plasmid instability, the *wbdR* gene was integrated into the chromosome of *B. abortus* vaccine candidates (BABΔwadC, BABΔppdK and BABΔwadCΔppdK). To this end, a technique of gene targeting with mini-Tn7, using the suicide plasmid pUC18R6KTmini-Tn7TKm (Llobet et al, Antimicrob Agents Chemother.,53(1):298-302, 2009) was used. This strategy relies on the stable Tn7 insertion in the region placed in the intergenic region between genes *glmS* (encoding the glucosamine- 6-phosphate synthetase) and *recG* (encoding a ATP-dependent DNA helicase) (Choi et al., Nat Methods., 2(6):443-8,2005; and Nat Protoc., 1(1):170-8,2006)
For tagging, *B. abortus* constructs were mated with donors *E. coli* S17λpir (carrying the pUC18R6KTmini-Tn7TKm-PwbdR) and *E. coli* SM10λpir pTNS2 (whose plasmid encodes the information for Tn7 transposition) with the assistance of *E. coli* HB101 pRK2013 (that allows the mobilization of plasmids). Bacteria in which integration occurred were selected on media containing kanamycin (BAB ΔwadCΔppdK::wbdR). To ensure that the insertion occurred immediately after the stop codon of *glmS*, PCRs were performed using oligonucleotides Tn7R-Tn7L specific for the ends of Tn7, and *glmS-* and *recG*-specific oligonuclotides. To confirm that a single insertion of the Tn7 had occurred, Southern-blotting was performed. This method of tagging also applies to other constructs.

### Intracellular multiplication assays

Bone marrow cells were isolated from femurs of 6-8-week-old C57B1/6 female mice and differentiated into dendritic cells (BMDC) as described previously (Inaba et al., J Exp Med, 176: 1693-1702, 1992) in the presence of de-complemented fetal calf serum (eurobio, France) and GM-CSF. Infections were performed by centrifuging the bacteria onto the differentiated cells (400×g for 10 min at 4°C) at a bacteria:cells ratio of 30:1 and incubated at 37°C (5% CO₂) for 30 min. Cells were then gently washed with culture medium to remove extracellular bacteria and incubated in medium supplemented with 50 µg/mL gentamicin for 1 h to kill any remaining extracellular bacteria. Thereafter, the antibiotic concentration was decreased to 10 µg/mL. To monitor *Brucella* intracellular survival, the infected cells were lysed with 0.1% (vol/vol) Triton X-100 in H₂O at 24h and 48h post-infection, and serial dilutions of the recovered lysates were performed before plating on to tryptic soy agar (in triplicate) to enumerate CFU.

The levels of IL-6, IL-12 p70 and TNF-α, were determined by enzyme-linked immunosorbent assays according to the manufacturer's instructions (ELISA kit from eBioscience) in the supernatants of BMDC at 24 hours post-infection (see above) with different *Brucella* wild type and mutant strains.

### Flow cytometry

At 24h post-infection with different wild-type and mutant *Brucella* strains (see above), BMDCs were collected and immediately stained for surface receptor expression prior to fixation with 3% paraformaldehyde. Cells were labelled with fluorochrome-conjugated antibodies specific for mouse CD11c:PE-Cy7 (clone N418), IA-IE:PE (MHC class II clone M5/114.15.2) (PE), CD86:FITC (Clone GL-1), CD40:AlexaFluor 647 (clone 3/23) and CD80:PE-Cy5 (clone 16-10A1), all from BioLegend. Labelled cells were then subjected to multi-colour cytometry using a FACScanto II (Bectin Dickinson) and the data was then analysed using FlowJo software, by first gating on the CD11c+ population prior to quantifying expression of receptors.

Figures 2 to 4 demonstrates that the double mutant is able to trigger an intense release of several inflammatory interleukins, particularly of IL6, IL12 and TNFα The specific combination of mutations in the ppdK and wadC genes in a bacterium according to the invention generates a strong immune response and thereby enhances the efficiency of a vaccine containing such mutant. The double mutant induces a significantly higher pro-inflammatory cytokine production than the simple ΔwadC mutant (regardless of the strain used).

Figure 1 further demonstrates that the dendritic cells are able to fully eliminate the double mutant at 48H after injection.

## Claims

1. A modified bacterium of the genus *Brucella* carrying an inactivated *wadC* gene and an inactivated *ppdK* gene, wherein said genes encode either a non-functional protein or no protein.

2. The modified bacterium according to claim 1, wherein said bacterium further expresses a heterologous enzyme that acylates the polysaccharide O side chains (O-PS) of the lipopolysaccharide (LPS).

3. The modified bacterium according to claim 2, wherein said heterologous enzyme acylates the perosamine of the O-PS.

4. The modified bacterium according to claim 2 or 3, wherein said heterologous enzyme transfers an acetyl group to the O-PS.

5. The modified bacterium according to any one of claims 2 to 4, wherein said heterologous enzyme is a N-acetyltansferase expressed by the wbdR gene of E.coli.

6. The modified bacterium according to any one of claims 1 to 5 wherein said bacterium belongs to a species selected from the group consisting of Brucella abortus, Brucella melitensis, Brucella suis, Brucella ovis, Brucella pinnipedialis, Brucella ceti, Brucella microti, and Brucella canis.

7. The modified bacterium according to claim 6 wherein the species of said bacterium is Brucella abortus or Brucella melitensis.

8. The modified bacterium according to claim 7 wherein the species of said bacterium is Brucella abortus.

9. The modified bacterium according to any one of claims 1 to 8 for use as a vaccine of the human or animal body.

10. The modified bacterium according to any one of claims 1 to 8 for use in a method for vaccinating a subject against a disease caused by a bacterium of the genus *Brucella.*

11. The modified bacterium for use according to claim 10 wherein said disease is brucellosis.

12. The modified bacterium for use according to any one of claim 10 or 11, wherein said subject is an animal selected from the group consisting of cattle, sheep, goats and swine.

## Patentansprüche

1. Ein modifiziertes Bakterium des Genus *Brucella,* das ein inaktiviertes *wadC*-Gen und ein inaktiviertes *ppdK-*Gen trägt, wobei diese Gene entweder ein nicht-funktionales Protein oder kein Protein kodieren.

2. Modifiziertes Bakterium nach Anspruch 1, wobei das Bakterium weiterhin ein heterologes Enzym exprimiert, das die Polysaccharid-O-Seitenketten (O-PS) des Lipopolysaccharids (LPS) acyliert.

3. Modifiziertes Bakterium nach Anspruch 2, wobei das heterologe Enzym das Perosamin des O-PS acyliert.

4. Modifiziertes Bakterium nach Anspruch 2 oder 3, wobei das heterologe Enzym eine Acetylgruppe auf das O-PS überträgt.

5. Modifiziertes Bakterium nach einem der Ansprüche 2 bis 4, wobei das heterologe Enzym eine N-Acetyltransferase, die durch das wbdR-Gen von E.coli exprimiert wird, ist.

6. Modifiziertes Bakterium nach einem der Ansprüche 1 bis 5, wobei das Bakterium zu einer Spezies, ausgewählt aus der Gruppe, bestehend aus Brucella abortus, Brucella melitensis, Brucella suis, Brucella ovis, Brucella pinnipedialis, Brucella ceti, Brucella microti und Brucella canis, gehört.

7. Modifiziertes Bakterium nach Anspruch 6, wobei die Spezies des Bakteriums Brucella abortus oder Brucella melitensis ist.

8. Modifiziertes Bakterium nach Anspruch 7, wobei die Spezies des Bakteriums Brucella abortus ist.

9. Modifiziertes Bakterium nach einem der Ansprüche 1 bis 8 zur Verwendung als Vakzine für den humanen oder Tierkörper.

10. Modifiziertes Bakterium nach einem der Ansprüche 1 bis 8 zur Verwendung in einem Verfahren zur Vakzinierung eines Subjekts gegen eine Erkrankung, die durch ein Bakterium des Genus *Brucella* verursacht wird.

11. Modifiziertes Bakterium zur Verwendung nach Anspruch 10, wobei die Erkrankung Brucellose ist.

12. Modifiziertes Bakterium zur Verwendung nach einem der Ansprüche 10 oder 11, wobei das Subjekt ein Tier, ausgewählt aus der Gruppe, bestehend aus Rindern, Schafen, Ziegen und Schweinen ist.

## Revendications

1. Bactérie modifiée du genre *Brucella* portant un gène *wadC* inactivé et un gène *ppdK* inactivé, dans laquelle lesdits gènes codent soit pour une protéine non fonctionnelle soit pour aucune protéine.

2. Bactérie modifiée selon la revendication 1, dans laquelle ladite bactérie exprime en outre une enzyme hétérologue qui acyle les chaînes latérales O-polysaccharides (O-PS) du lipopolysaccaride (LPS).

3. Bactérie modifiée selon la revendication 2, dans laquelle ladite enzyme hétérologue acyle la pérosamine de l'O-PS.

4. Bactérie modifiée selon la revendication 2 ou 3, dans laquelle ladite enzyme hétérologue transfère un groupe acétyle à l'O-PS.

5. Bactérie modifiée selon l'une quelconque des revendications 2 à 4, dans laquelle ladite enzyme hétérologue est une N-acétyltransférase exprimée par le gène *wbdR d'E. coli.*

6. Bactérie modifiée selon l'une quelconque des revendications 1 à 5 dans laquelle ladite bactérie appartient à une espèce sélectionnée dans le groupe consistant en *Brucella abortus*, *Brucella melitensis*, *Brucella suis*, *Brucella ovis*, *Brucella pinnipedialis*, *Brucella ceti*, *Brucella microti*, et *Brucella canis.*

7. Bactérie modifiée selon la revendication 6 dans laquelle l'espèce de ladite bactérie est *Brucella abortus* ou *Brucella melitensis.*

8. Bactérie modifiée selon la revendication 7 dans laquelle l'espèce de ladite bactérie est *Brucella abortus.*

9. Bactérie modifiée selon l'une quelconque des revendications 1 à 8 pour son utilisation comme un vaccin du corps humain ou animal.

10. Bactérie modifiée selon l'une quelconque des revendications 1 à 8 pour son utilisation dans un procédé de vaccination d'un sujet contre une maladie provoquée par une bactérie du genre *Brucella.*

11. Bactérie modifiée pour son utilisation selon la revendication 10 dans laquelle ladite maladie est la brucellose.

12. Bactérie modifiée pour son utilisation selon l'une quelconque des revendications 10 ou 11, dans laquelle ledit sujet est un animal sélectionné dans le groupe consistant en un bovin, un ovin, un caprin et un porcin.


### SEQUENCE LISTING

<110> INSERM
<120> MODIFIED BACTERIA FOR IMPROVED VACCINES AGAINST BRUCELLOSIS
<130> BEP140372
<160> 4
<170> PatentIn version 3.5
<210> 1
   <211> 20
   <212> DNA
   <213> Artificial Sequence
<220>
   <223> Primer
<400> 1
   ctcccgattc atttttcacg 20
<210> 2
   <211> 19
   <212> DNA
   <213> Artificial Sequence
<220>
   <223> Primer
<400> 2
   tgctcatttc agccaggtt 19
<210> 3
   <211> 39
   <212> DNA
   <213> Artificial Sequence
<220>
   <223> Primer
<400> 3
   aacctggctg aaatgagcac gggtctcgac tatgtgtcc 39
<210> 4
   <211> 20
   <212> DNA
   <213> Artificial Sequence
<220>
   <223> Primer
<400> 4
   tcaacgcatc aaagcagaag 20